(19) Europäisches Patentamt
European Patent Office
Office européen des brevets

(11) Veröffentlichungsnummer: **0 033 105**
**A2**

# (12) EUROPÄISCHE PATENTANMELDUNG

(21) Anmeldenummer: **81100325.0**

(22) Anmeldetag: **17.01.81**

(51) Int. Cl.³: **C 07 D 495/04**
A 01 N 47/18
//(C07D495/04, 333/00, 231/00)

(30) Priorität: **29.01.80 DE 3003019**

(43) Veröffentlichungstag der Anmeldung:
**05.08.81 Patentblatt 81/31**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Maurer, Fritz, Dr.**
**Roeberstrasse 8**
**D-5600 Wuppertal(DE)**

(72) Erfinder: **Hammann, Ingeborg, Dr.**
**Belfortstrasse 9**
**D-5000 Koeln(DE)**

(72) Erfinder: **Homeyer, Bernard, Dr.**
**Obere Strasse 28**
**D-5090 Leverkusen(DE)**

(54) **N,N-Dimethyl-O-(4,6-dihydro-2H-thieno(3,4-c)pyrazol-3-yl)-carbaminsäureester sowie deren 5-Oxide und 5,5-Dioxide, Verfahren zu deren Herstellung und ihre Verwendung in Schädlingsbekämpfungsmitteln.**

(57) Gegenstand der vorliegenden Erfindung sind

(1) neue N,N-Dimethyl-0-(4,6-dihydro-2H-thieno[3,4-c]-pyrazol-3-yl)-carbaminsäureester sowie deren 5-Oxide und 5,5-Dioxide der Formel I

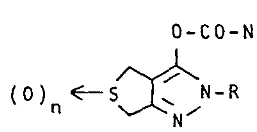

(1)

in welcher

R für gegebenenfalls substituiertes Alkyl, Alkenyl, Alkinyl, Cycloalkyl, Aralkyl oder Aryl steht und

n für Null, 1 oder 2 steht;

(2) ein Verfahren zu ihrer Herstellung, dadurch gekennzeichnet, daß man 4,6-Dihydro-2H-thieno [3,4-c] 3-hydroxy-pyrazole bzw. deren 5-Oxide oder 5,5-Dioxide der Formel II

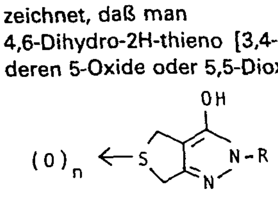

(11)

carbamoyliert, oder im Falle von Verbindungen der Formel (I), in welcher n für 1 oder 2 steht, Verbindungen der Formel (I), in welcher n für Null steht, oxidiert.

Gegenstand der Erfindung sind ferner

(3) neue 4,6-Dihydro-2H-thieno [3,4-c] 3-hydroxy-pyrazole sowie deren 5-Oxide und 5,5-Dioxide der Formel II

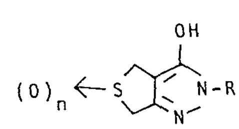

(11)

und

(4) ein Verfahren zu ihrer Herstellung

Die neuen N,N-Dimethyl-0-(4,6-dihydro-2H-thieno[3,4-c]-pyrazol-3-yl)-carbaminsäureester sowie deren 5-Oxide und 5,5-Dioxide der Formel (I) zeichnen sich durch hohe pestizide, insbesondere insektizide Wirksamkeit aus.

EP 0 033 105 A2

BAYER AKTIENGESELLSCHAFT
Zentralbereich
Patente, Marken und Lizenzen

5090 Leverkusen, Bayerwerk
Rt/W
Typ Ib

N,N-Dimethyl-O-(4,6-dihydro-2H-thieno[3,4-c]pyrazol-3-yl)-carbaminsäureester sowie deren 5-Oxide und 5,5-Dioxide, Verfahren zu deren Herstellung und ihre Verwendung in Schädlingsbekämpfungsmitteln

Die Erfindung betrifft neue N,N-Dimethyl-O-(4,6-dihydro-2H-thieno[3,4-c] pyrazol-3-yl)-carbaminsäureester sowie deren 5-Oxide und 5,5-Dioxide, mehrere Verfahren zu deren Herstellung und ihre Verwendung in Schädlingsbekämpfungsmitteln, insbesondere in Insektiziden.

Es ist bekannt, daß bestimmte N,N-Dimethyl-O-pyrazolyl-carbaminsäureester, wie z.B. N,N-Dimethyl-O-(1-phenyl-3-methyl-pyrazol-5-yl)- und N,N-Dimethyl-O-(1-isopropyl-3-methyl-pyrazol-5-yl)-carbaminsäureester, insektizide Eigenschaften aufweisen (vergleiche CH-PS 282 655).

Die insektizide Wirkung dieser bekannten Verbindungen ist jedoch, insbesondere bei niedrigen Wirkstoffkonzentrationen und Aufwandmengen, nicht immer zufriedenstellend.



BAD ORIGINAL

Gegenstand der vorliegenden Erfindung sind

(1) neue N,N-Dimethyl-O-(4,6-dihydro-2H-thieno[3,4-c]-pyrazol-3-yl)-carbaminsäureester sowie deren 5-Oxide und 5,5-Dioxide der Formel I

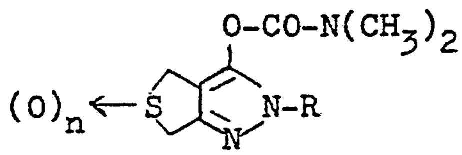

(I)

in welcher

R für gegebenenfalls substituiertes Alkyl, Alkenyl, Alkinyl, Cycloalkyl, Aralkyl oder Aryl steht und

n für Null, 1 oder 2 steht;

(2) Verfahren zur Herstellung der neuen Verbindungen der Formel I, dadurch gekennzeichnet, daß man

4,6-Dihydro-2H-thieno[3,4-c] 3-hydroxy-pyrazole bzw. deren 5-Oxide oder 5,5-Dioxide der Formel II

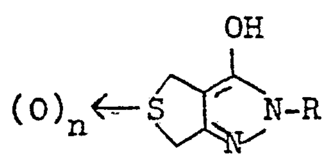

(II)

in welcher

R und n die oben angegebenen Bedeutungen haben,



BAD ORIGINAL

carbamoyliert, oder im Falle von Verbindungen der Formel (I), in welcher n für 1 oder 2 steht, Verbindungen der Formel (I), in welcher n für Null steht, oxidiert.

Gegenstand der Erfindung sind ferner

(3) neue 4,6-Dihydro-2H-thieno[3,4-c] 3-hydroxy-pyrazole sowie deren 5-Oxide und 5,5-Dioxide der Formel II

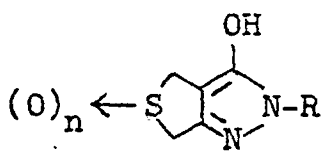

(II)

in welcher

R für gegebenenfalls substituiertes Alkyl, Alkenyl, Alkinyl, Cycloalkyl, Aralkyl oder Aryl steht und

n für Null, 1 oder 2 steht, und

(4) ein Verfahren zur Herstellung von Verbindungen der Formel (II), dadurch gekennzeichnet, daß man 4-Oxo-tetrahydrothiophen-3-carbonsäureester der Formel III

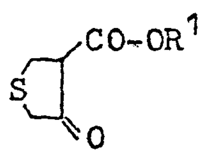

(III)

in welcher

$R^1$ für Alkyl mit 1 bis 4 Kohlenstoffatomen steht,



BAD ORIGINAL

mit Hydrazinderivaten der Formel IV

$$H_2N\text{-}NH\text{-}R \qquad (IV)$$

in welcher

R die oben angegebene Bedeutung hat,

gegebenenfalls in Gegenwart eines Säureakzeptors und gegebenenfalls unter Verwendung eines Verdünnungsmittels umsetzt und gegebenenfalls die Produkte nach üblichen Methoden oxidiert.

Die neuen N,N-Dimethyl-O-(4,6-dihydro-2H-thieno[3,4-c]-pyrazol-3-yl)-carbaminsäureester sowie deren 5-Oxide und 5,5-Dioxide der Formel (I) zeichnen sich durch hohe pestizide, insbesondere insektizide Wirksamkeit aus.

Überraschenderweise zeigen die erfindungsgemäßen Verbindungen der Formel (I) eine erheblich höhere insektizide Wirkung als aus dem Stand der Technik bekannte Verbindungen analoger Konstitution und gleicher Wirkungsrichtung.

Gegenstand der Erfindung sind vorzugsweise Verbindungen der Formel (I), in welcher

R für gegebenenfalls durch Cyano substituiertes $C_1$-$C_5$-Alkyl oder für $C_3$-$C_6$-Cycloalkyl steht und

n für Null, 1 oder 2 steht.



BAD ORIGINAL

Die Carbamoylierung zur Herstellung der neuen Verbindungen der Formel I, kann dadurch erfolgen, daß man

(2 a) 4,6-Dihydro-2H-thieno[3,4-c] 3-hydroxy-pyrazole bzw. deren 5-Oxide oder 5,5-Dioxide der Formel II

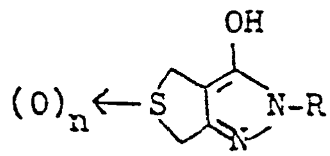 (II)

in welcher

R und n die oben angegebenen Bedeutungen haben,

mit N,N-Dimethylcarbaminsäurechlorid, gegebenenfalls in Gegenwart eines Säureakzeptors und gegebenenfalls unter Verwendung eines Verdünnungsmittels umsetzt, oder

(2 b) Verbindungen der Formel II (oben) mit Phosgen und anschließend mit Dimethylamin, gegebenenfalls in Gegenwart eines Säureakzeptors und gegebenenfalls unter Verwendung eines Verdünnungsmittels umsetzt.

(2 c) Die Oxidation der Verbindungen der Formel (I), in welcher

n für O steht, zu Verbindungen in welchen n für 1 steht, kann dadurch erfolgen, daß Verbindungen der Formel (I), in welcher n für Null steht, mit wenigstens angenähert der äquimolaren Menge Wasserstoffperoxid, gegebenenfalls unter Verwendung eines Verdünnungsmittels umgesetzt werden.



BAD ORIGINAL

(2. d) Die Oxidation der Verbindungen der Formel (I), in welcher n für O steht, zu Verbindungen in welchen n für 2 steht, kann dadurch erfolgen, daß Ver-Verbindungen der Formel (I), in welcher n für Null steht, mit wenigstens zwei Moläquivalenten m-Chlorperbenzoesäure, gegebenenfalls in Gegenwart eines Verdünnungsmittels umgesetzt werden.

Verwendet man beispielsweise bei den Verfahren (2 a) und (2 b) 2-Isobutyl-4,6-dihydro-2H-thieno-[3,4-c]-3-hydroxypyrazol und bei den Verfahren (c) und (d) N,N-Dimethyl-O-(2-propyl-4,6-dihydro-2H-thieno[3,4-c] pyrazol-3-yl)-carbaminsäureester als Ausgangsstoffe, so können deren Reaktionen durch folgende Formelschemata skizziert werden:

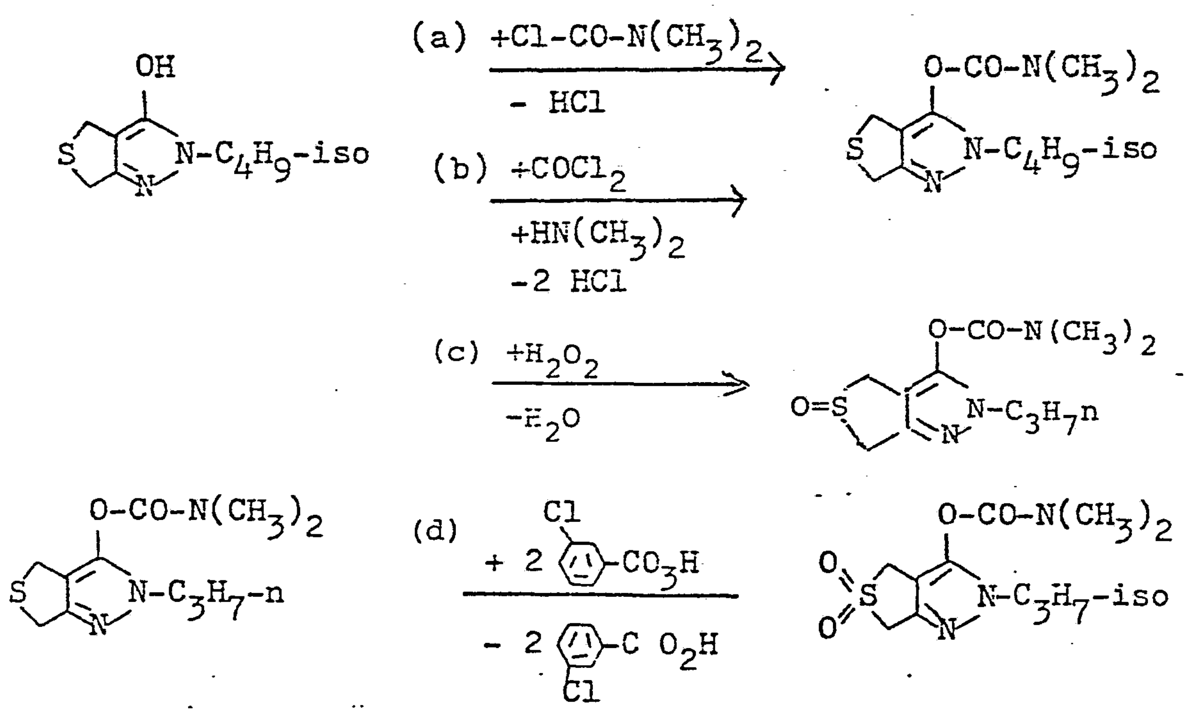




BAD ORIGINAL

Die bei den Verfahren (2 a) und (2 b) als Ausgangsstoffe zu verwendenden 4,6-Dihydro-2H-thieno[3,4-c]-3-hydroxy-pyrazole sowie deren 5-Oxide und 5,5-Dioxide sind durch Formel (II) definiert. Vorzugsweise stehen darin

R für gegebenenfalls durch Cyano substituiertes $C_1$-$C_5$-Alkyl oder für $C_3$-$C_6$-Cycloalkyl und

n für Null.

Als Beispiele für die Verbindungen der Formel (II) seien genannt:
2-Methyl-, 2-Ethyl-, 2-n-Propyl-, 2-iso-Propyl-, 2-n-Butyl-, 2-iso-Butyl-, 2-sek.-Butyl-, 2-tert-Butyl-, 2-n-Pentyl-, 2-iso-Pentyl-, 2-sek.-Pentyl-, 2-tert-Pentyl-, 2-(1-ethyl-propyl)-, 2-(2-cyano-ethyl)-, 2-Cyclopropyl-, 2-Cyclopentyl- und 2-Cyclohexyl-4,6-dihydro-2H-thieno-[3,4-c] 3-hydroxy-pyrazol.

Die Verbindungen der Formel (II) sind noch nicht in der Literatur beschrieben. Man erhält sie, wie oben unter (4) dargelegt, indem man Verbindungen der Formel (III) (oben), wie z.B. 4-Oxo-tetrahydrothiophen-3-carbonsäuremethylester, mit Hydrazinderivaten der Formel IV (oben), in welcher R vorzugsweise für gegebenenfalls durch Cyano substituiertes $C_1$-$C_5$-Alkyl oder für $C_3$-$C_6$-Cycloalkyl steht, gegebenenfalls in Gegenwart eines Säureakzeptors wie z.B. Natriummethylat und gegebenenfalls unter Verwendung eines Verdünnungsmittels, wie z.B. Methanol, bei Temperaturen zwischen 1o und 1oo°C umsetzt. Zur Aufarbeitung wird mit Wasser verdünnt, gegebenenfalls das organische Lösungsmittel abdestilliert, die verbleibende Lösung schwach angesäuert und

das kristallin abgeschiedene Produkt durch Filtration isoliert.

Als Beispiele für die Vorprodukte der Formel (IV) seien genannt:
Methyl-, Ethyl-, n-Propyl-, iso-Propyl-, n-Butyl-, iso-Butyl-, sek-Butyl-, tert.-Butyl-, n-Pentyl-, iso-Pentyl-, sek.-Pentyl-, tert-Pentyl-, 1-Ethyl-propyl-, 2-Cyano-ethyl-, Cyclopropyl-, Cyclopentyl- und Cyclohexyl-hydrazin.

Die Vorprodukte der Formeln (III) und (IV) sind bekannt.

Die bei den Verfahren (2 c) und (2 d) als Ausgangsstoffe zu verwendenden N,N-Dimethyl-O-(4,6-dihydro-2H-thieno-[3,4-c] pyrazol-3-yl)-carbaminsäureester sind durch die Formel (I) mit der Maßgabe, daß n für Null steht, definiert.

Vorzugsweise steht darin

R für gegebenenfalls durch Cyano substituiertes $C_1$-$C_5$-Alkyl oder für $C_3$-$C_6$-Cycloalkyl.

Die Verfahren (2 a) bis (2 d) werden vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol,



BAD ORIGINAL

Ether, wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Verfahren (2 c) wird vorteilhaft unter Verwendung aliphatischer Carbonsäuren, wie z.B. Ameisensäure, Essigsäure oder Propionsäure als Verdünnungsmitteln durchgeführt.

Verfahren (2 a) und (2 b) werden im allgemeinen unter Verwendung von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die erfindungsgemäßen Verfahren werden im allgemeinen bei Temperaturen zwischen o und 15o°C durchgeführt. Bevorzugt wird für Verfahren (2 a) der Temperaturbereich zwischen 2o und 1oo°C, für Verfahren (2 b), (2 c) und (2 d) der Bereich zwischen o und 5o°C. Die Umsetzungen werden im allgemeinen bei Normaldruck durchgeführt.



BAD ORIGINAL

Zur Durchführung von Verfahren (2 a) bzw. (2 b) werden auf 1 Mol Hydroxy-pyrimidin in der Formel (II) zwischen 1,o und 1,3, vorzugsweise zwischen 1,o und 1,15 Mol N,N-Dimethylcarbaminsäurechlorid bzw. Phosgen und Dimethylamin eingesetzt. Die Umsetzung wird im allgemeinen in einem Verdünnungsmittel in Gegenwart eines Säureakzeptors durchgeführt. Nach Ablauf der Reaktion wird mit Wasser verdünnt und mit einem mit Wasser nicht mischbaren organischen Lösungsmittel, wie z.B. Toluol, extrahiert. Die organische Phase wird getrocknet, filtriert und vom Filtrat wird das Lösungsmittel unter vermindertem Druck abdestilliert.

Bei Verfahren (2 c) werden die Reaktionskomponenten bevorzugt in äquimolaren Mengen eingesetzt. Bei Verwendung von Verdünnungsmitteln, die mit Wasser mischbar sind, werden diese nach Reaktionsende im Vakuum abdestilliert. Der Rückstand wird dann in einem mit Wasser nicht mischbaren Lösungsmittel, wie z.B. Methylenchlorid, gelöst und nach üblichen Methoden, z.B. durch Waschen, Trocknen, Filtrieren und Abdestillieren des Lösungsmittels aufgearbeitet.

Bei Verfahren (2 d) wird die als Oxidationsmittel verwendete m-Chlor-perbenzoesäure gewöhnlich im Überschuß eingesetzt, und zwar zwischen 2 und 3 Mol je Mol N,N-Dimethyl-O-(2H-thieno[3,4-c]pyrazol-3-yl)-carbaminsäureester. Die Umsetzung wird im allgemeinen in einem mit Wasser nicht mischbaren Lösungsmittel durchgeführt. Nach Ablauf der Reaktion wird neutral gewaschen, getrocknet, filtriert und das Lösungsmittel im Vakuum abdestilliert.



BAD ORIGINAL

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Verbindungen nach dem Abdestillieren des Lösungsmittels in fester Form anfallen, werden sie durch Umkristallisieren gereinigt. Zur Charakterisierung dient dann der Schmelzpunkt.



BAD ORIGINAL

## Herstellungsbeispiele

**Beispiel 1:**

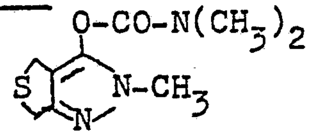

Zu einer Mischung aus 7,8 g (o,o5 Mol) 2-Methyl-4,6-dihydro-2H-thieno[3,4-c] 3-hydroxy-pyrazol, 8,4 g (o,o6 Mol) Kaliumcarbonat, 2oo ml Acetonitril und 5,4 g (o,o5 Mol) N,N-Dimethyl-carbaminsäurechlorid wird 12 Stunden bei 5o°C gerührt. Nach Zugabe von 2oo ml Wasser extrahiert man mit 3oo ml Toluol. Die organische Phase wird über Natriumsulfat getrocknet und im Vakuum eingedampft. Zurück bleiben lo g (88% der Theorie) N,N-Dimethyl-O-(2-methyl-4,6-dihydro-2H-thieno[3,4-c] pyrazol-3-yl)-carbaminsäureester in Form eines beigen Pulvers mit dem Schmelzpunkt 71°C.

**Beispiel 2:**

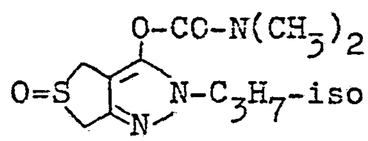

Eine Lösung von 12,7 g (o,o5 Mol) N,N-Dimethyl-O-(2-isopropyl-4,6-dihydro-2H-thieno[3,4-c] pyrazol-3-yl)-carbaminsäureester in 5o ml Eisessig wird bei 5-lo°C mit 3,4 g (o,o5 Mol) 5o%igem Wasserstoffperoxid versetzt. Man rührt das Gemisch 6 Stunden bei Raumtemperatur nach und destilliert dann das Lösungsmittel im Vakuum ab. Der Rückstand wird in loo ml Methylenchlorid gelöst und mit einer Lösung von lo g Kaliumcarbonat in 15 ml Wasser gewaschen. Die organische Phase wird abgetrennt und über Natriumsulfat getrocknet. Dann destilliert man das Lösungsmittel im Vakuum ab. Man erhält so 12,5 g (92% der Theorie) N,N-Dimethyl-O-(2-isopropyl-4,6-di-



BAD ORIGINAL

hydro-2H-thieno-[3,4-c] pyrazol-3-yl)carbaminsäureester--5-oxid in Form hellbrauner Kristalle mit dem Schmelzpunkt 92°C.

Baispiel 3:

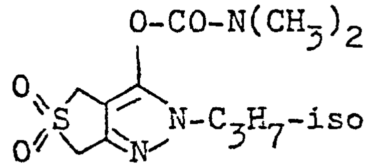

Zu einer Lösung von 12,7 g (o,o5 Mol) N,N-Dimethyl-O-(2-iso-propyl-4,6-dihydro-2H-thieno[3,4-c] pyrazol-3-yl)-carbaminsäureester in 5o ml Chloroform tropft man bei 5°C eine Lösung von 21,3 g m-Chlorperbenzoesäure in 15o ml Chloroform. Das Gemisch wird über Nacht bei Raumtemperatur nachgerührt und dann filtriert. Man wäscht das Filtrat mit lo ml konzentrierter Kaliumcarbonatlösung und trocknet es über Natriumsulfat. Dann wird das Lösungsmittel im Vakuum abgezogen. Zurück bleiben 13,8 g (96% der Theorie) N,N-Dimethyl-O-(2-iso-propyl-4,6-dihydro-2H-thieno[3,4-c] pyrazol-3-yl)-carbaminsäureester-5,5-dioxid in Form eines beigen Pulvers mit dem Schmelzpunkt 123°C.

Analog einem der Beispiele 1 bis 3 können die folgenden Verbindungen der Formel

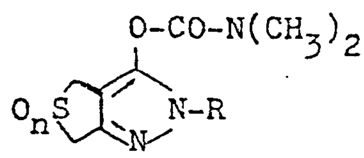

hergestellt werden:



BAD ORIGINAL

| Beispiel Nr. | R | n | Ausbeute (% der Theorie) | Physikal. Daten (Brechungsindex; Schmelzpunkt °C) |
|---|---|---|---|---|
| 4 | $C_3H_7$-iso | 0 | 85 | $n_D^{24}$:1,531o |
| 5 | $C_2H_5$ | 0 | | |
| 6 | $C_4H_9$-sek. | 0 | | |
| 7 | 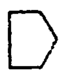 | 0 | | |
| 8 | $C_2H_5$ | 1 | | |
| 9 | $C_2H_5$ | 2 | | |
| 1o | $C_4H_9$-sek. | 1 | | |
| 11 | $C_4H_9$-sek. | 2 | | |
| 12 | 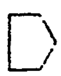 | 1 | | |
| 13 | 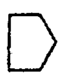 | 2 | | |
| 14 | $CH_3$ | 1 | | |
| 15 | $CH_3$ | 2 | | |
| 16 | $CH_2$-$CH_2$-CN | 0 | | |

| Beispiel Nr. | R | n | Ausbeute (% der Theorie) | Physikal.Daten (Brechungsindex; Schmelzpunkt °C) |
|---|---|---|---|---|
| 17 | $CH_2$-$CH_2$-CN | 1 | | |
| 18 | $CH_2$-$CH_2$-CN | 2 | | |
| 19 | 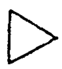 | 0 | | |
| 2o | 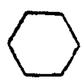 | 0 | | |
| 21 | $CH(C_2H_5)_2$ | 0 | | |
| 22 | $C_4H_9$-tert. | 0 | | |

Die als Ausgangsstoffe zu verwendenden 4,6-Dihydro-2H-thieno[3,4-c]3-hydroxy-pyrazole können z.B. wie folgt hergestellt werden:

Beispiel a: 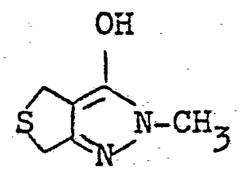

Zu einer Lösung von 4,6 g (o,1 Mol) Methylhydrazin und 16 g (o,1 Mol) 4-Oxo-tetrahydrothiophen-3-carbonsäure-methylester (Darstellung s. R.B. Woodward u.R.H.Eastman, J.Amer.Chem.Soc. 68 (1946), S. 2232) in 1oo ml Methanol gibt man o,1 Mol einer Lösung von Natriummethylat in Methanol und kocht das Gemisch 2 Stunden unter Rückfluß. Dann gibt man 1oo ml Wasser zu und destilliert das Methanol im Vakuum ab. Die zurückbleibende Lösung wird durch Zugabe von konzentrierter Salzsäure auf pH 5 eingestellt, dann saugt man das ausgefallene Produkt ab und wäscht mit wenig Eiswasser nach. Man erhält auf diese Weise 8 g (51% der Theorie) 2-Methyl-4,6-dihydro-2H-thieno[3,4-c]3-hydroxy-pyrazol in Form eines farblosen Pulvers mit dem Schmelzpunkt 21o°C.

In analoger Weise können die folgenden Verbindungen der Formel

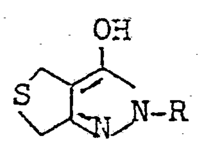

hergestellt werden:



BAD ORIGINAL

| Beispiel Nr. | R | Ausbeute (% der Theorie) | Schmelzpunkt (°C) |
| --- | --- | --- | --- |
| b | $C_3H_7$-iso | 79 | 191 |
| c | $C_2H_5$ | | |
| d | $C_4H_9$-sek. | | 2o4 |
| e | 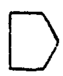 | | 211 |
| f | $CH_2$-$CH_2$-CN | | |
| g | 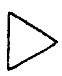 | | |
| h | 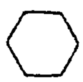 | | |
| i | $CH(C_2H_5)_2$ | | |

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.



BAD ORIGINAL

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,



BAD ORIGINAL

Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..



BAD ORIGINAL

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff



BAD ORIGINAL

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.



BAD ORIGINAL

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.



BAD ORIGINAL

Beispiel A

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 4,2.



BAD ORIGINAL

## Beispiel B

Doralis-Test (systemische Wirkung)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 4,2,3.



BAD ORIGINAL

Beispiel C

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4,1,2,3.



BAD ORIGINAL

Patentansprüche

1. N,N-Dimethyl-O-(4,6-dihydro-2H-thieno[3,4-c]-pyrazol-3-yl)-carbaminsäureester sowie deren 5-Oxide und 5,5-Dioxide der Formel I

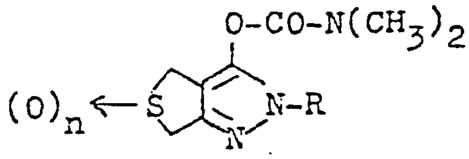

(I)

in welcher

R für gegebenenfalls substituiertes Alkyl, Alkenyl, Alkinyl, Cycloalkyl, Aralkyl oder Aryl steht und

n für Null, 1 oder 2 steht.

2. Verfahren zur Herstellung der N,N-Dimethyl-O-(4,6-dihydro-2H-thieno[3,4-c]-pyrazol-3-yl)-carbaminsäureester sowie deren 5-Oxide und 5,5-Dioxide der Formel I, dadurch gekennzeichnet, daß man 4,6-Dihydro-2H-thieno[3,4-c] 3-hydroxy-pyrazole bzw. deren 5-Oxide oder 5,5-Dioxide der Formel II

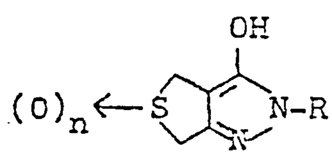

(II)

in welcher

R und n die oben angegebenen Bedeutungen haben,

carbamoyliert oder im Falle von Verbindungen der Formel (I), in welcher n für 1 oder 2 steht, Verbindungen der Formel (I), in welcher n für Null oxidiert.



BAD ORIGINAL

3. 4,6-Dihydro-2H-thieno/¯3,4-c/ 3-hydroxy-pyrazole sowie deren 5-Oxide und 5,5-Dioxide der Formel II

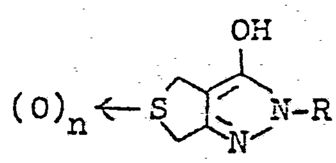

(II)

in welcher

R für gegebenenfalls substituiertes Alkyl, Alkenyl, Alkinyl, Cycloalkyl, Aralkyl oder Aryl steht und

n für Null, 1 oder 2 steht.

4. Verfahren zur Herstellung von Verbindungen der Formel (II), dadurch gekennzeichnet, daß man 4-Oxo-tetrahydrothiophen-3-carbonsäureester der Formel III

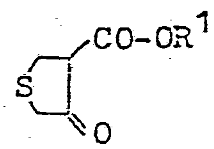

(III)

in welcher

$R^1$ für Alkyl mit 1 bis 4 Kohlenstoffatomen steht,

mit Hydrazinderivaten der Formel IV

$$H_2N\text{-}NH\text{-}R \qquad (IV)$$

in welcher

R die oben angegebene Bedeutung hat,
gegebenenfalls in Gegenwart eines Säureakzeptors und gegebenenfalls unter Verwendung eines Verdünnungsmittels umsetzt und gegebenenfalls die Produkte nach üblichen Methoden oxidiert.



BAD ORIGINAL

4. Schädlingsbekämpfungsmittel, gekennzeichnet durch einen Gehalt an mindestens einem N,N-Dimethyl-O-(4,6-dihydro-2H-thieno/¯3,4-c7-pyrazol-3-yl)-carbaminsäureester oder einem davon abgeleiteten 5-Oxid- und 5,5-Dioxid der Formel (I).

5. Verwendung von N,N-Dimethyl-O-(4,6-dihydro-2H-thieno-/¯3,4-c7-pyrazol-3-yl)-carbaminsäureester sowie deren 5-Oxiden und 5,5-Dioxiden der Formel (I) zur Bekämpfung von Schädlingen.

6. Verfahren zur Bekämpfung von Schädlingen, dadurch gekennzeichnet, daß man N,N-Dimethyl-O-(4,6-dihydro-2H-thieno/¯3,4-c7-pyrazol-3-yl)-carbaminsäureester sowie deren 5-Oxide und 5,5-Dioxide der Formel (I) auf Schädlinge und/oder ihren Lebensraum einwirken läßt.

7. Verfahren zur Herstellung von Schädlingsbekämpfungsmitteln, dadurch gekennzeichnet, daß man N,N-Dimethyl-O-(4,6-dihydro-2H-thieno/¯3,4-c7-pyrazol-3-yl)-carbaminsäureester sowie deren 5-Oxide und 5,5-Dioxide der Formel (I) mit Streckmitteln und/oder oberflächenaktiven Mitteln vermischt.



BAD ORIGINAL